Europäisches Patentamt

(19) European Patent Office  (11) Publication number: **0 028 987**

Office européen des brevets  **B1**

(12) **EUROPEAN PATENT SPECIFICATION**

(45) Date of publication of patent specification: **27.06.84**  (51) Int. Cl.³: **A 61 K 39/29, C 12 N 7/00**

(21) Application number: **80401613.7**

(22) Date of filing: **12.11.80**

(54) Process for the in vitro culture of hepatitis B virus.

(30) Priority: **13.11.79 US 93658**

(43) Date of publication of application:
**20.05.81 Bulletin 81/20**

(45) Publication of the grant of the patent:
**27.06.84 Bulletin 84/26**

(84) Designated Contracting States:
**AT BE CH DE FR GB IT LI LU NL SE**

(56) References cited:
**US - A - 4 164 566**

**CHEMICAL ABSTRACTS, vol. 91, October 8, 1979, no. 15, ref. 118970y, page 197, COLUMBUS, OHIO (US), MONTJARDINO J. et al.: "Polypeptide of HBsAg excreted by a human hepatoma cell line"**
**BIOLOGICAL ABSTRACTS, vol. 63, 1977, ref. 52500, PHILA. (US), MACNAB G.M. et al.: "Hepatitis B surface antigen produced by a human heptoma cell line"**
**BIOLOGICAL ABSTRACTS, vol. 70, 1980, PHILA. (US), COPELAND JENNIFER A. et al.: "Hepatitis B surface antigen production as a growth cycle related terminal event in PLC/PRF/5 hepatoma cells"**

(73) Proprietor: **MERCK & CO. INC.**
**126, East Lincoln Avenue P.O. Box 2000 Rahway New Jersey 07065 (US)**

(72) Inventor: **Hilleman, Maurice R.**
**4107 Fields Drive**
**Lafayette Hill Pennsylvania 19444 (US)**
Inventor: **Keller, Paul M.**
**201 Oberlin Terrace Lansdale**
**Pennsylvania 19446 (US)**
Inventor: **McAleer, William J.**
**717 Marietta Drive**
**Ambler Pennsylvania 19002 (US)**
Inventor: **Provost, Philip J.**
**473 Landis Road**
**Harleysville Pennsylvania 19438 (US)**

(74) Representative: **Martin, Jean-Jacques et al, Cabinet REGIMBEAU 26, Avenue Kléber F-75116 Paris (FR)**

(56) References cited:
**CHEMICAL ABSTRACTS, vol. 92, no. 11, March 17, 1980, ref. 92540b, page 442, COLUMBUS, OHIO (US), MARION, PATRICIA L. et al.: "Polypeptides of hepatitis B virus surface antigen produced by a hepatoma cell line"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

NATURE, vol. 282, December 6, 1979, no. 5739, BASINGSTOKE (US), DAVID P. ADEN et al.: "Controlled synthesis of HBsAg in a differentiated human liver carcinoma-derived cell line" pages 615-616
CHEMICAL ABSTRACTS, vol. 93, no. 17, October 27, 1980, ref. 164212s, page 337, COLUMBUS, OHIO (US), DUBOIS, Marie Françoise et al.: "Excretion of hepatitis B surface antigen particles from mouse cells transformed with cloned viral DNA"
NATURE, vol. 282, December 6, 1979, JACINTA SKELLY et al.: "Hepatitis B surface antigen produced by a human hepatoma cell line" pages 617-618

## Description

Heretofore the only source of hepatitis B surface antigen ($HB_sAg$) has been blood or tumor cell culture from human carriers of hepatitis B disease. Such human carriers are difficult to locate with the result that the needed blood is scarce and expensive.

It is an object of the present invention to provide a method for obtaining hepatitis B virus which does not require blood from human carriers. Another object is to provide a method for the *in vitro* cell culture of hepatitis B virus. These and other objects of the present invention will be apparent from the following description.

*In vitro* cell cultures derived from liver of a hepatitis B infected primate produce hepatitis B surface and core antigens over an extended period of time.

The present invention relates to the *in vitro* cell culture propagation of hepatitis B virus and, more particularly, to *in vitro* cell culture propagation of hepatitis B virus in cell cultures derived from the liver of a hepatitis B infected primate.

According to the present invention a clinical specimen containing hepatitis B virus, e.g. blood, isolated virus, liver, plasma, saliva or serum from a human or non-human infected with hepatitis B, is used as the inoculum. The non-human primate may be any species susceptible to hepatitis B disease, e.g., baboon, chimpanzee, gorilla, orangutan, or rhesus monkey. The clinical specimen may be used to inoculate a non-human primate. The inoculation is preferably by intravenous injection. At from 42 to 90 days post inoculation, typically at about 70 days post inoculation, a liver specimen from the infected animal is used to establish an in vitro cell culture. Alternatively, a liver specimen from a human patient suffering from hepatitis B may be used.

The infected liver cell culture is incubated in the presence of a suitable nutrient medium. By a nutrient medium is meant a cell culture medium which permits growth of cells *in vitro*. Some specific nutrient media are, for example, Medium 199, Morgan et al., Proc. Soc. Exp. Biol. & Med., 73:1—8, 1950; Basal Medium Eagle, Eagle, Science, 122, 501—504, 1955; In Vitro, Vol. 6, No. 1970; Dulbecco's Modified Eagle's Medium, Dulbecco et al., Virology, 8, 396, 1959; Smith et al, J. Viol., 12, 185—196, 1960; In Vitro, Vol. 6, No. 2, 1970; Minimum Essential Medium (Eagle), Science, 130, 432 (1959), RPMI Media, Moore et al., 199, 519—524, 1967; In vitro, Vol. 6, No. 2, 1970, Williams Medium E, Exp. Cell Res., Vol. 69, 106—112, 1971; or Ham's F12, 1965 PNAS, Vol. 53, 288; in the presence of serum, e.g., chimp serum or fetal calf serum. The incubation is preferably effected in the presence of one or more hormones which inhibit growth of fibroblasts. Such hormones are, for instance, insulin, glucagon and 9 - fluoro - 11$\beta$,17,21 - trihydroxy - 16$\alpha$ - methylpregna - 1,4 - diene - 3,20 - dione. These substances are generally employed at a concentration of from $10^{-5}$ to $10^{-7}$ M. The incubation preferably is carried out at from 32 to 39°C, most preferably at about 35°C in an atmosphere of air containing about 5% $CO_2$ by volume. A vigorous outgrowth of hepatocyte-like epithelial cells usually begins within 7 days. Nine days post planting hepatitis B core antigen ($HB_cAg$) is detected by fluorescent antibody or radioimmune assay (RIA) while $HB_sAg$ is detected by RIA. These cultures continue to produce heptitis B core and surface antigen for a period of at least 150 days.

Optionally, but preferably, the hepatitis B virus is serially transmitted through at least one susceptible primate prior to removal of the liver specimen for *in vitro* cell culture.

The following example illustrates the present invention without, however, limiting the same thereto.

## Example

A chimpanzee is inoculated intravenously with 1 ml of a 1:1,000 dilution of a human hepatitis B carrier plasma. Liver tissue is removed 43 or more days following inoculation and finely minced by chopping, the mince is washed and planted in cell culture medium (Williams Medium E with 0 to 20% animal serum, $10^{-6}$ M insulin, dexamethasone, and glucagon). Cultures in 25 cm² flasks are refed with 2 to 3 ml of culture media 2 to 3 times per week. Incubation is at 35°C on a rocker platform in a gas atmosphere of 5% $CO_2$ and air. Within 7 days a vigorous outgrowth of hepatocyte-like epithelial cells begins. This description of chimpanzee liver cell culture is pertinent whether the liver is obtained from normal or hepatitis B infected animals.

$HB_cAg$ is detected by fluorescent antibody at nine days post planting in the nuclei or hepatocyte-like cells which have grown out from the mince. It is possible to specifically block this fluorescence by convalescent human hepatitis B serum but not by pre-illness serum from the same patient, and by post-immunized but not pre-immunized guinea pig serum.

$HB_sAg$ is also detected by RIA in the culture fluids either going from a negative to a positive or maintaining a positive state after repeated dilution due to refeeding. Uninfected chimp livers do not develop this effect. $HB_sAg$ and $HB_cAg$ continue to be produced for at least 150 days.

## Claims

1. A process for the *in vitro* cell culture of hepatitis B virus, characterized by the fact that it comprises incubating at a temperature of from 32 to 39°C in the presence of a nutrient medium a cell culture of liver tissue removed from a human or non-human primate infected with hepatitis B, and continuing the incubation until the presence of hepatitis B viral antigens is detected in the cell culture.

2. A process according to claim 1, characterized by the fact that hepatitis B viral antigens are harvested from the cell culture.

3. A process according to one of claims 1 and 2, characterized by the fact that the incubation takes place in the presence of a substance which inhibits growth of fibroblasts.

4. A process according to claim 3, characterized by the fact that said substance is insulin, glucagon or 9 - fluoro - 11β,17,21 - trihydroxy - 16α - methyl - pregna - 1,4 - diene - 3,20 - dione.

5. A process according to one of claims 3 and 4, characterized by the fact that said substance is present in a concentration of from $10^{-5}$ to $10^{-7}$ mole.

6. A process according to one of claims 1 to 5, characterized by the fact that the incubation takes place at 35°C.

7. A process according to one of claims 1 to 6, characterized by the fact that the hepatitis B virus is serially transmitted through one or more susceptible primates prior to removal of liver tissue for cell culture.

**Patentansprüche**

1. Verfahren zur Zellkultur von Hepatitis-B-Virus in vitro, dadurch gekennzeichnet, daß man bei einer Temperatur von 32 bis 39°C, in Anwesenheit eines Nährmediums, eine Zellkultur von Lebergewebe, entnommen aus einem menschlichen oder nicht-menschlichen Primaten, der mit Hepatitis B infiziert ist, kultiviert und die Inkubation fortsetzt, bis die Anwesenheit von Hepatitis-B-Virus-antigenen in der Zellkultur festgestellt wird.

2. Verfahren nach Anspruch 1, dadurch gekennzeichnet, daß die Hepatitis-B-Virus-antigene aus der Zellkultur geerntet werden.

3. Verfahren nach einem der Ansprüche 1 und 2, dadurch gekennzeichnet, daß die Inkubation in Anwesenheit einer Substanz erfolgt, die das Wachstum von Fibroblasten inhibiert.

4. Verfahren nach Anspruch 3, dadurch gekennzeichnet, daß die Substanz Insulin, Glucagon oder 9 - Fluor - 11β,17,21 - trihydroxy - 16α - methyl - pregna - 1,4 - dien - 3,20 - dion ist.

5. Verfahren nach einem der Ansprüche 3 und 4, dadurch gekennzeichnet, daß die Substanz in einer Konzentration von $10^{-5}$ bis $10^{-7}$ Mol vorhanden ist.

6. Verfahren nach einem der Ansprüche 1 bis 5, dadurch gekennzeichnet, daß die Inkubation bei 35°C erfolgt.

7. Verfahren nach einem der Ansprüche 1 bis 6, dadurch gekennzeichnet, daß das Hepatitis-B-Virus serienmäßig durch einen oder mehrere anfällige Primaten übertragen wird, bevor Lebergewebe zur Zellkultur entnommen wird.

**Revendications**

1. Un procédé pour la culture cellulaire in vitro du virus de l'hépatite B caractérisé par le fait qu'il comprend l'incubation à une température de 32 à 39°C en présence d'un milieu nutritif, d'une culture de cellules de tissu hépatique prélevé à un primate humain ou non-humain infecté par l'hépatite B, et la poursuite de l'incubation jusqu'à ce que la présence des antigènes viraux de l'hépatite B soit détectée dans la culture cellulaire.

2. Un procédé selon la revendication 1, caractérisé par le fait que les antigènes viraux de l'hépatite B sont recueillis à partir de la culture cellulaire.

3. Un procédé selon l'une des revendications 1 et 2, caractérisé par le fait que l'incubation s'effectue en présence d'une substance qui inhibe le développement des fibroblastes.

4. Un procédé selon la revendication 3, caractérisé par le fait que ladite substance est l'insuline le glucagon, ou la 9 - fluoro - 11β,17,21 - trihydroxy - 16α - methyl - pregna - 1,4 - diène - 3,20 - dione.

5. Un procédé selon l'une quelconque des revendications 3 et 4, caractérisé par le fait que ladite substance est présente à une concentration de $10^{-5}$ à $10^{-7}$ mole.

6. Un procédé selon l'une des revendications 1 à 5, caractérisé par le fait que l'incubation s'effectue à 35°C.

7. Un procédé selon l'une des revendications 1 à 6, caractérisé par le fait qu'on réalise des passages successifs du virus de l'hépatite B sur un ou plusieurs primates sensibles avant de prélever le tissu hépatique pour la culture cellulaire.